(19) Europäisches Patentamt
European Patent Office
Office européen des brevets

(11) Veröffentlichungsnummer: **0 268 963**
**A1**

(12) **EUROPÄISCHE PATENTANMELDUNG**

(21) Anmeldenummer: 87116840.7

(22) Anmeldetag: 14.11.87

(51) Int. Cl.4: C07D 487/04 , A61K 31/395

(30) Priorität: 28.11.86 DE 3640715

(43) Veröffentlichungstag der Anmeldung: 01.06.88 Patentblatt 88/22

(84) Benannte Vertragsstaaten: AT BE CH DE ES FR GB IT LI NL SE

(71) Anmelder: **BAYER AG**
**Konzernverwaltung RP Patentabteilung**
**D-5090 Leverkusen 1 Bayerwerk(DE)**

(72) Erfinder: **Häbich, Dieter, Dr.**
**Krummacher Strasse 82**
**D-5600 Wuppertal 1(DE)**
Erfinder: **Hartwig, Wolfgang, Dr.**
**Pahlkestrasse 3**
**D-5600 Wuppertal 1(DE)**
Erfinder: **Metzger, Karl Georg, Dr.**
**Pahlkestrasse 75**
**D-5600 Wuppertal 1(DE)**
Erfinder: **Zeiler, Hans-Joachim, Dr.**
**Elsbeeker Strasse 46**
**D-5620 Velbert 15(DE)**

(54) **Benzazolylthio-carbapenem-Antibiotika.**

(57) Die Erfindung betrifft Amino-substituierte Benzoxazolylthio-, Benzthiazolylthio-sowie Benzimidazolylthio-carbapenem-Antibiotika der allgemeinen Formel I

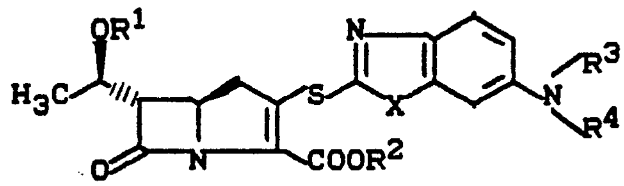

(I)

in welcher $R^1$ bis $R^4$ und X die in der Beschreibung angegebene Bedeutung haben, Verfahren zu ihrer Herstellung und ihre Verwendung als Arzneimittel.

EP 0 268 963 A1

## Benzazolylthio-carbapenem-Antibiotika

Die Erfindung betrifft Amino-substituierte Benzoxazolylthio-, Benzthiazolylthio-sowie Benzimidazolylthio-carbapenem-Antibiotika, Verfahren zu ihrer Herstellung und ihre Verwendung als Arzneimittel.

Es ist bekannt, daß sich Carbapeneme vom Thienamycin-Typ [T. Kametani, Heterocycles 17, 463 (1982)] durch gute antibakterielle Wirkung auszeichenen, die zum Großteil auch methicillin-und oxacillin-resistente Staphylokokken erfaßt [B.G. Christensen et al. in A. G. Brown & S. M. Roberts (eds), Recent Advances in the Chemistry of β-Lactam-Antibiotics, Royal Society of Chemistry, Special Publication No. 52, 86 (1985)]. Gegen bestimmte Staphylokokken und Enterokokken weisen Verbindungen wie Thienamycin und Imipenem jedoch antibakterielle Schwächen auf; außerdem sind viele Derivate dieser Verbindungen als Monosubstanz instabil gegen den Abbau durch die Peptidasen der Niere, speziell Dehydropeptidase (I) (DHP-I) [H.Kropp et al., Antimicrob. Agents., Chemother. 22, 62 (1982)]. Dieses Enzym ist verantwortich für die metabolische Inaktivierung von Carbapenemen und für die Bildung von toxischen Metaboliten.

Es wurden nun Benzazolylthio-carbapenem-Antibiotika der allgemeinen Formel (I)

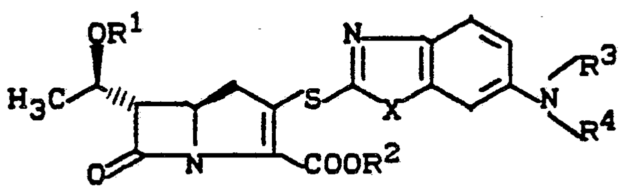 (I)

in welcher

$R^1$ -für Wasserstoff oder
-für eine Hydroxyschutzgruppe steht,
$R^2$ -für Wasserstoff oder
-für eine Carboxyschutzgruppe oder
-für einen in vivo abspaltbaren Esterrest steht,
$R^3$ -für Wasserstoff oder
-für $C_1$-$C_{10}$-Alkyl, $C_6$-$C_{12}$-Aryl oder $C_7$-$C_{14}$-Aralkyl steht,
$R^4$ -für Wasserstoff oder
-für $C_1$-$C_{10}$-Alkyl, $C_6$-$C_{12}$-Aryl oder $C_7$-$C_{14}$-Aralkyl steht, oder
-für $C_1$-$C_{10}$-Alkylsulfonyl, $C_6$-$C_{12}$-Arylsulfonyl oder $C_7$-$C_{14}$-Aralkylsulfonyl steht, oder
-für eine Aminoschutzgruppe steht, oder
-für eine Gruppe der Formel

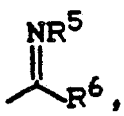, 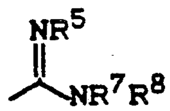 oder 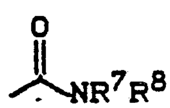 steht,

worin

$R^5$, $R^6$, $R^7$ und $R^8$ gleich oder verschieden sind und - Wasserstoff, $C_1$-$C_{10}$-Alkyl, $C_6$-$C_{12}$-Aryl oder $C_7$-$C_{14}$-Aralkyl bedeuten

und

X - für O, S, NH oder $NCH_3$ steht

und deren Salze gefunden.

Aminoschutzgruppe im Rahmen der oben angegebenen Definition steht im allgemeinen für eine in der β-Lactam-Chemie übliche Aminoschutzgruppe. Bevorzugt zu nennen sind beispielsweise:

Vinyl, Allyl, tert.Butoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, 4-Methoxybenzyloxycarbonyl, Formyl, Benzoyl, Acetyl, Ethylcarbonyl, Chloracetyl, Trichloracetyl, Trifluoroacetyl, Methyloxycarbonyl, Allyloxycarbonyl, Trimethylsilyl, Triethylsilyl, Triphenylsilyl, tert.Butyl-dimethylsilyl, Methyldiphenylsilyl, 2,4-Dimethoxybenzyl, 2,4-Dimethoxybenzyloxycarbonyl, 4-Methoxybenzyl, 4-(Methoxymethyloxy)phenyl, Bis-(4-Methoxyphenyl)methyl, tert.Butoxycarbonylmethyl, Allyloxycarbonylmethyl, Methoxymethyl, Methylthiomethyl, Methoxyethoxymethyl, [2-(Trimethylsilyl)ethoxy]methyl oder 2-(Methylthiomethoxy)ethoxycarbonyl.

Hydroxyschutzgruppe im Rahmen der oben angegebenen Definition steht im allgemeinen für eine in der $\beta$-Lactam-Chemie übliche Hydroxyschutzgruppe. Bevorzugt zu nennen sind beispielsweise:

Trimethylsilyl, Triethylsilyl, Triisopropylsilyl, tert.-Butyl-dimethylsilyl, Triphenylsilyl, Trimethylsilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, tert.Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]methyl, 2-(Methylthiomethoxy)ethoxycarbonyl, Tetrahydropyranyl oder Benzoyl.

Carboxyschutzgruppe im Rahmen der oben angegebenen Definition steht für die in der $\beta$-Lactam-Chemie üblichen Carboxyschutzgruppe, Bevorzugt sind leicht abspaltbare Gruppen zu nennen wie zum Beispiel: Methyl, Ethyl, tert.Butyl, Decyl, 2-Chlorethyl, 2,2,2-Trichlorethyl, Cyanoethyl, Diphenylmethyl, Triphenylmethyl, Acetoxymethyl, Allyl, Benzyl, 4-Methoxyphenyl, 4-Nitrobenzyl, 2-Nitrobenzyl, 4-Methoxybenzyl, 2,4-Dimethoxybenzyl, Trimethylsilylethyl, Trimethylsilyl, tert.Butyl-dimethylsilyl, Acetonyl, 1-Phenoxyethyl oder 2-Methyl-2-propenyl.

Steht $R^2$ für einen in vivo leicht abspaltbaren Esterrest so sind hiermit pharmazeutisch verträgliche Esterreste gemeint, die in vivo leicht zu freien Carboxylgruppen ($R^2$ = H) hydrolysiert werden.

Solche Esterreste sind auf dem $\beta$-Lactam-Gebiet gut bekannt. In den meisten Fällen bessern sie die Absorptionseigenschaften der $\beta$-Lactam-Verbindungen. Außerdem sollte der Rest $R^2$ von solcher Art sein, daß er einer Verbindung der Formel (I) pharmazeutisch annehmbare Eigenschaften verleiht und bei Spaltung in vivo pharmazeutisch annehmbare Fragmente freisetzt.

Beispiele für solche Gruppen befinden sich in der DE-OS 2 517 316. Bevorzugte in vivo abspaltbare Estergruppen sind die der Folgenden Formeln:

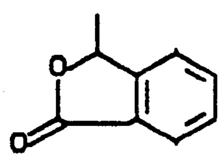 , 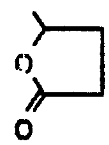 , 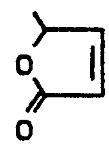 ,

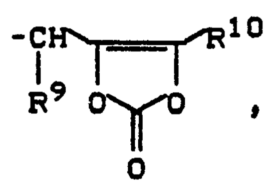 , 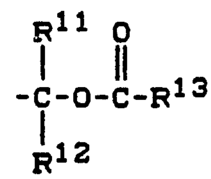 oder

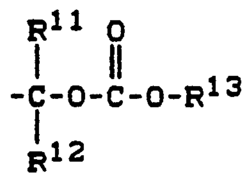

worin

$R^9$ und $R^{10}$ gleich oder verschieden sind und

-für Wasserstoff, Phenyl oder

-für $C_1$-$C_4$-Alkyl, bevorzugt für Methyl stehen,

$R^{11}$ und $R^{12}$ gleich oder verschieden sind und

-für Wasserstoff oder

-für $C_1$-$C_4$-Alkyl, bevorzugt Methyl stehen

und

$R^{13}$ -für $C_1$-$C_6$-Alkyl, bevorzugt für $C_1$-$C_4$-Alkyl steht.

Bevorzugt sind Verbindungen der allgemeinen Formel (I) in welcher

$R^1$ -für Wasserstoff steht, oder

-für eine Hydroxyschutzgruppe aus der Reihe Trimethylsilyl, Triethylsilyl, Triisopropylsilyl, tert.-Butyldimethylsilyl, Triphenylsilyl, Trimethylsilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, tert.Butoxycarbonyl, Allyloxycarbonyl, 4-Methoxybenzyl, 4-Methoxybenzyloxycarbonyl, Formyl, Acetyl, Trichloracetyl, 2,2,2,-Trichlorethoxycarbonyl,

2,4-Dimethoxybenzyloxycarbonyl, Methoxymethyl oder Methoxyethoxymethyl steht,
$R^2$ -für Wasserstoff steht, oder
-für Methyl, Ethyl, tert.Butyl, 2-Chlorethyl, 2,2,2-Trichlorethyl, Cyanoethyl, Diphenylmethyl, Triphenylmethyl, Acetoxymethyl, Allyl, Benzyl, 4-Methoxyben zyl, 2,4-Dimethoxybenzyl, 1-Phenoxyethyl, 2-Methyl-2-propenyl, 4-Nitrobenzyl, 2-Nitrobenzyl, Trimethylsilylethyl oder tert.Butyl-dimethylsilylethyl steht, oder
-für einen Rest der Formel

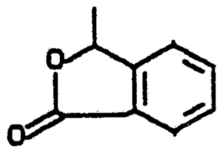 , 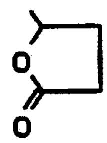 , 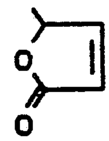 ,

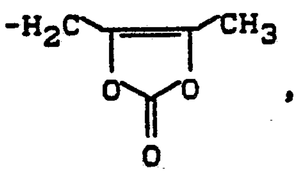 ,

$-CH_2-OCO-C(CH_3)_3$, $-CH(CH_3)-OCOOC_2H_5$ oder $-CH_2-OCOCH_3$ steht,
$R^3$ -für Wasserstoff oder
-für $C_1$-$C_6$-Alkyl steht, oder
-für Phenyl oder Benzyl steht,
$R^4$ -für Wasserstoff steht, oder
-für $C_1$-$C_6$-Alkyl steht, oder
-für Phenyl oder Benzyl steht, oder
-für $C_1$-$C_4$-Alkylsulfonyl, Phenylsulfonyl oder Benzylsulfonyl steht, oder
-für eine Aminoschutzgruppe aus der Reihe Allyl, tert.Butoxycarbonyl, Benzyl, Benzyloxycarbonyl, 4-Nitrobenzyl, 4-Nitrobenzyloxycarbonyl, 4-Methoxybenzyloxycarbonyl, Formyl, Acetyl, Trichloracetyl, 4-Methoxyphenyl, 4-Methoxybenzyl, 2,4-Dimethoxybenzyl, 2-Nitrobenzyloxycarbonyl, tert.Butyl-dimethylsilyl, Methyldiphenylsilyl, Trimethylsilyl oder Trifluoracetyl steht, oder
-für eine Gruppe der Formel

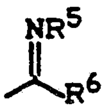 , 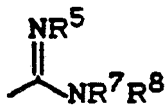 oder 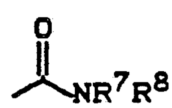 steht,

worin
$R^5$, $R^6$, $R^7$ und $R^8$ gleich oder verschieden sind und - Wasserstoff oder $C_1$-$C_6$-Alkyl, Phenyl oder Benzyl bedeuten
und
X - für O oder S steht, oder
-für NH oder $NCH_3$ steht
und deren Salze.

Besonders bevorzugt sind solche Verbindungen der allgemeinen Formel (I)
in welcher
$R^1$ -für Wasserstoff steht, oder
-für Trimethylsilyl, Triphenylsilyl, Trimethylsilylethoxycarbonyl, tert.Butyl-dimethylsilyl, 2-Nitrobenzyloxycarbonyl, 4-Nitrobenzyloxycarbonyl, Allyloxycarbonyl oder Formyl steht,
$R^2$ -für Wasserstoff steht, oder
-für Methyl, Ethyl, tert.Butyl, 2,2,2-Trichlorethyl, Allyl, Acetoxymethyl, 4-Nitrobenzyl, 2-Nitrobenzyl, 4-Methoxybenzyl, Benzyl oder Trimethylsilylethyl steht, oder
-für einen Rest der Formel

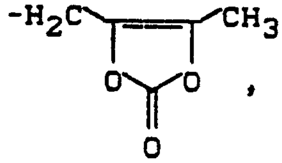,

$-CH(CH_3)-OCOOC_2H_5$ oder $-CH_2-OCO-C(CH_3)_3$ steht,
$R^3$ -für Wasserstoff, $C_1$-$C_4$-Alkyl oder Phenyl steht,
$R^4$ -für Wasserstoff oder
-für $C_1$-$C_4$-Alkyl, Phenyl, tert.Butyloxycarbonyl, Benzyloxycarbonyl, Acetyl, Formyl oder Ethylcarbonyl steht, oder
-für eine Gruppe der Formel

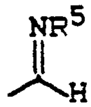 , 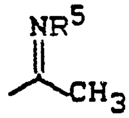 , 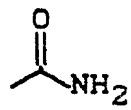 oder 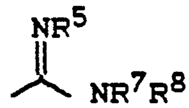 steht,

worin
$R^5$, $R^7$ und $R^8$ gleich oder verschieden sind und - Wasserstoff, Methyl oder Phenyl bedeuten,
und
X - für S, NH oder $NCH_3$ steht
und deren Salze.

Neben den in den Beispielen aufgeführten erfindungsgemäßen Verbindungen sind folgende Verbindungen der allgemeinen Formel (I) und deren Salze insbesondere bevorzugt:

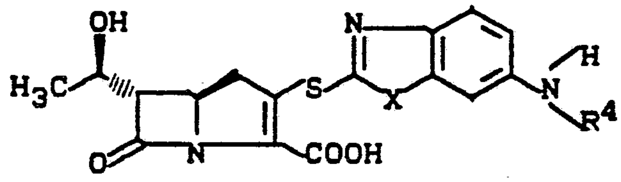

| X | $R^4$ | X | $R^4$ |
|---|---|---|---|
| S | 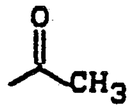 | O | 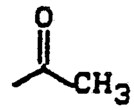 |
| S | 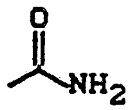 | O | 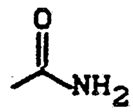 |
| S | 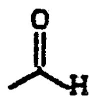 | O | 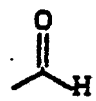 |
| S | 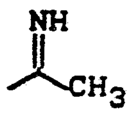 | O | 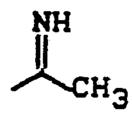 |
| S | 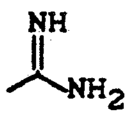 | O | 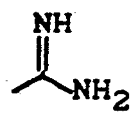 |
| S | 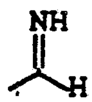 | O | 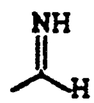 |
| S | 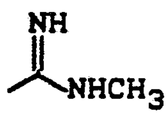 | O | 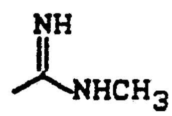 |
| S | 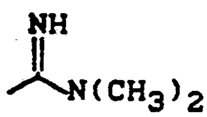 | O | 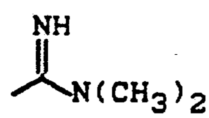 |
| S | 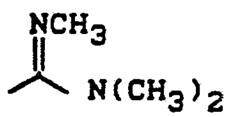 | O | 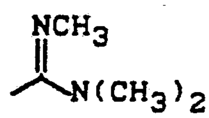 |

| X | $R^4$ | X | $R^4$ |
|---|---|---|---|
| NH | 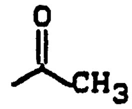 | $NCH_3$ | 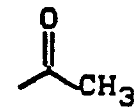 |
| NH | 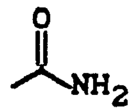 | $NCH_3$ | 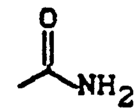 |
| NH | 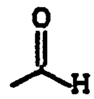 | $NCH_3$ | 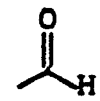 |
| NH | 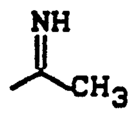 | $NCH_3$ | 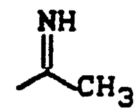 |
| NH | 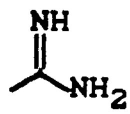 | $NCH_3$ | 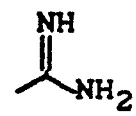 |
| NH | 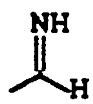 | $NCH_3$ | 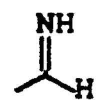 |
| NH | 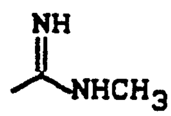 | $NCH_3$ | 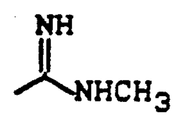 |
| NH | 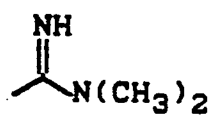 | $NCH_3$ | 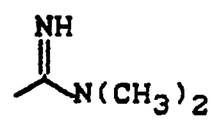 |
| NH | 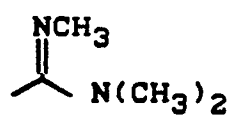 | $NCH_3$ | 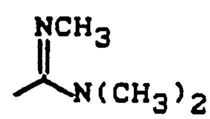 |

Die erfindungsgemäßen Verbindungen der allgemeinen Formel (I) können als freie Säuren, als Ester, als innere Salze

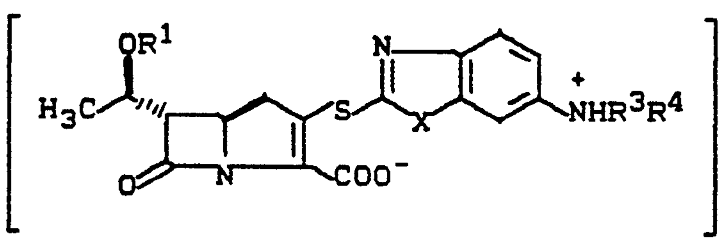 innere Salze

oder als nicht toxische physiologisch verträgliche Salze mit einem Gegenkation

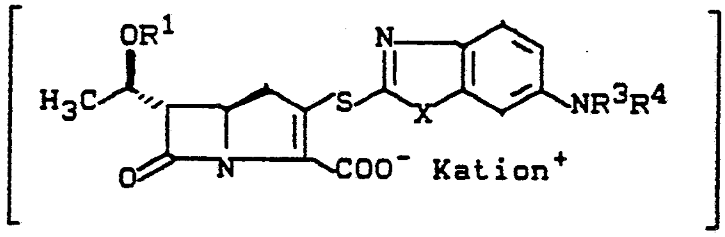

Salz mit Gegenkation

vorliegen.

Als Gegenkationen sind bevorzugt Alkali-oder Erdalkali-Kationen wie beispielsweise Natrium-, Kalium-, Magnesium-oder Calciumionen zu nennen, oder Aluminium-oder Ammoniumionen, sowie nicht toxische substituierte Ammoniumionen aus Aminen wie Diniedrigalkylamine, Triniedrigalkylamine, Procain, Dibenzylamin, N,N'-Dibenzylethylendiamin, N-Benzyl-$\beta$-phenyl-ethylamin, N-Methyl-morpholin, N-Methylmorpholin, 1-Ephenamin, Dihydroabiethylamin, N,N'-Bis-Dihydroabiethylethylendiamin, N-Niedrigalkylpiperidin und andere Amine, die zur Bildung von Salzen von $\beta$-Lactam-Verbindungen verwendet werden können.

Die erfindungsgemäßen Verbindungen besitzen mehrere asymmetrische Kohlenstoffatome und können somit in mehreren stereochemischen Formen existieren. Die Erfindung umfaßt die Isomerengemische ebenso wie die einzelnen Stereoisomeren. Bevorzugte Verbindungen der Formel (I) sind solche mit 5R, 6S, 8S-Konfiguration:

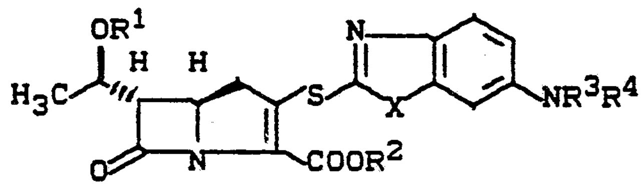

Darüberhinaus wurde ein Verfahren zur Herstellung der erfindungsgemäßen Benzazolylthio-Carbapenemantibiotika der allgemeinen Formel (I) und deren Salze gefunden,
das dadurch gekennzeichnet ist, daß man
Ketoester der allgemeinen Formel (II)

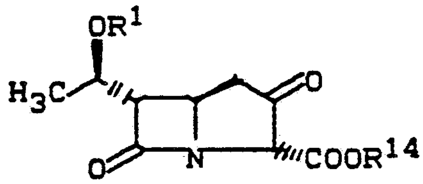

(II)

in welcher
$R^1$ -die angegebene Bedeutung hat und
$R^{14}$ -für eine Carboxyschutzgruppe oder
-für einen in vivo abspaltbaren Esterrest steht und
Thiole der allgemeinen Formel (III)

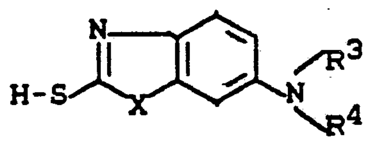

(III)

in welcher
$R^3$, $R^4$ und X die oben angegebene Bedeutung haben,
in inerten Lösemitteln in Gegenwart von Basen mit Hilfsstoffen umsetzt,
gegebenenfalls Schutzgruppen abspaltet
und gegebenenfalls die gewünschten Salze herstellt oder die Salze in die freien Verbindungen überführt.

Verwendet man als Ausgangsstoffe (2R,5R,6S)-3,7-Dioxo-6-[(1R)-1-hydroxyethyl]-1-azabicyclo[3.2.0]-

heptan-2-carbonsäure-p-nitrobenzylester und 6-Amino-2-mercapto-benzthiazol, so läßt sich das Verfahren durch folgendes Schema verdeutlichen:

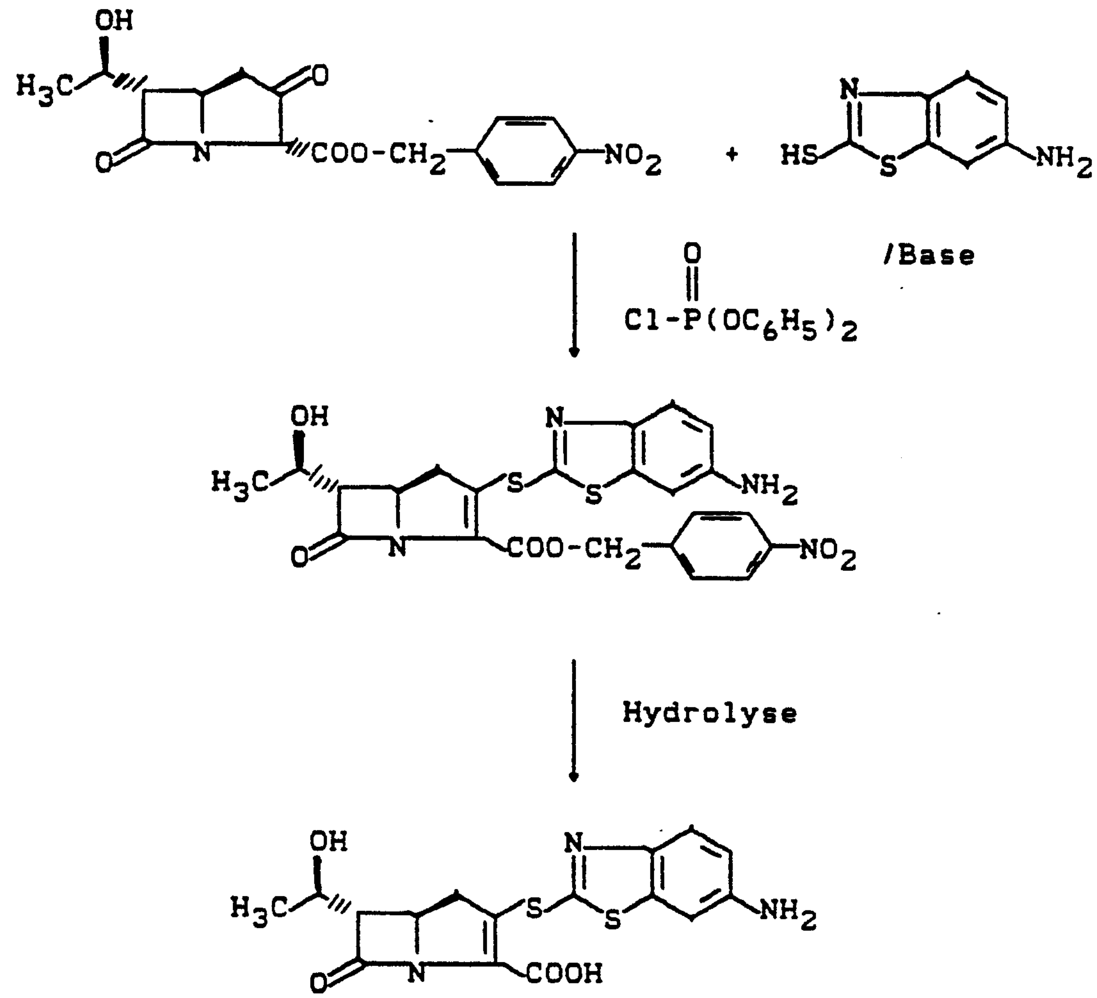


Als Lösemittel eignen sich hierbei alle organischen Lösemittel, die sich unter den Reaktionsbedingungen nicht verändern. Hierzu gehören bevorzugt Ether wie Diethylether, Dioxan, Tetrahydrofuran, Glykoldimethylether oder Butylmethylether, oder Kohlenwasserstoffe wie Benzol, Toluol, Xylol, Hexan, oder Erdölfraktionen, oder Halogenkohlenwasserstoffe wie Dichlormethan, Trichlormethan, Tetrachlormethan, 1,1,2-Trichlorethan, Dichlorethylen oder Trichlorethylen, Chlorbenzol oder Dichlorbenzol, oder Amide wie Dimethylformamid, Dimethylacetamid, Hexamethylphosphorsäure triamid, 1,3-Dimethyl-2-imidazolidinon (DMI), N,N'-Dimethyl-propylenharnstoff (DMPU), oder Aceton, Essigester, Sulfolan, Dimethylsulfoxid oder Acetonitril. Ebenso ist es möglich, Gemische der genannten Lösemittel einzusetzen.

Als Basen eignen sich die üblichen organischen Basen. Hierzu gehören bevorzugt Trialkylamine wie beispielsweise Trimethylamin, Triethylamin, Tripropylamin, Tributylamin oder Ethyldiisopropylamin, oder tertiäre organische Basen wie Pyridin, Dimethylaminopyridin, Picolin, Lutidin, N-Methylmorpholin, N-Methylpiperidin, 1,5-Diazabicyclo[5.4.0]-undec-5-en (DBU) oder 1,5-Diazabicyclo[4.3.0]non-3-en (DBN).

Als Hilfsstoffe werden im allgemeinen Stoffe eingesetzt, die die Ketoester der allgemeinen Formel (II) zu Verbindungen der allgemeinen Formel (IV)

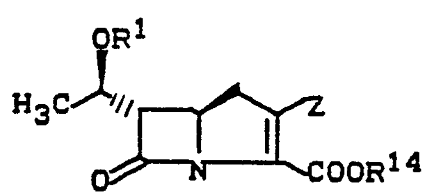

(IV)

in welcher

$R^1$ und $R^{14}$ die oben angegebene Bedeutung haben und
Z - für eine Abgangsgruppe aus der Reihe

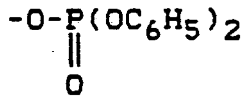 , $-OSO_2CF_3$, 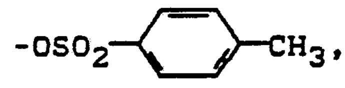

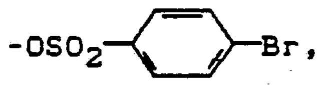, bevorzugt 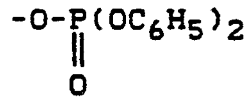 steht,

überführen.

Hierzu gehören bevorzugt Säurehalogenide oder Säureanhydride von 4-Toluolsulfonsäure, 4-Bromphenylsulfonsäure, Trifluormethansulfonsäure, oder Diphenylchlorphosphat. Besonders bevorzugt wird Diphenylchlorphosphat verwendet.

Die Reaktion kann in einem Schritt ohne Isolierung der Zwischenprodukte durchgeführt werden. Es hat sich jedoch als günstig erwiesen, die Zwischenprodukte der allgemeinen Formel (IV) zu isolieren.

Bevorzugt wird das Verfahren durchgeführt, indem man die Zwischenprodukte in einem geeigneten Lösemittel mit dem entsprechenden Thiol, gegebenenfalls in Anwesenheit von Basen umsetzt.

Ebenso ist es möglich, das Thiol (III) in Form seiner Salze einzusetzen oder das entsprechende Thiolat durch Zugabe von Base in der Reaktionsmischung herzustellen.

Die Base wird im allgemeinen in einer Menge von 1 bis 3 Mol, bevorzugt von 1 bis 1,5 Mol bezogen auf 1 Mol des Ketoesters eingesetzt. Das Thiol wird im allgemeinen in einer Menge von 1 bis 4 Mol, bevorzugt von 1 bis 2 Mol bezogen auf 1 Mol des Ketoesters eingesetzt. Erzeugt man aus dem Thiol in der Reaktionslösung das Thiolat so wird weitere Base in Mengen von 1 bis 5 Mol, bevorzugt von 1 bis 2 Mol bezogen auf 1 Mol Thiol eingesetzt.

Die Umsetzung wird im allgemeinen in einem Temperaturbereich von -80°C bis +60°C, bevorzugt von -50°C bis +40°C durchgeführt. Im allgemeinen führt man die Reaktion bei Normaldruck durch. Es ist aber ebenso möglich, bei Unterdruck oder bei Überdruck zu arbeiten.

Die als Ausgangsstoffe eingesetzten Thiole der allgemeinen Formel (III) sind bekannt oder können nach bekannten Methoden hergestellt werden [G.Bloeckingen et al. Tschechische Patentanmeldung 168 746/15.4.1977; CA 88, 37786(1978); V.Sutoris et al, Chem.Zvest. 27, 698(1973); CA 80,95814(1974); J.Roy et al. Indian J.Chem.,Sect B, 14B(7),536(1976)).

Die als Ausgangsstoffe eingesetzten Ketoester der allgemeinen Formel (II) sind bekannt oder können nach bekannten Methoden hergestellt werden [T.N. Salzmann et al., J. Am. Chem. Soc. 102, 6163 (1980)].

Die erfindungsgemäßen Verbindungen haben überraschenderweise im Vergleich zu konventionellen Carbapenemantibiotika wie beispielsweise Thienamycin oder Imipenem, besonders gegen zahlreiche Staphylokokken und Enterokokken eine verbesserte antibakterielle Aktivität. Darüberhinaus zeigen die erfindungsgemäßen Carbapeneme der Formel (I) metabolische Stabilität gegenüber dem Enzym Dehydropeptidase (I) (DHP I) und sind damit anderen Verbindungen dieser Substanzklasse in der Verträglichkeit überlegen.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human-und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept.

agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen-und Gelenkinfektionen, zystische Fibrose, Hautinfektionen, postoperative Wundinfektionen, Abszesse, Phlegmone, Wundinfektionen, infizierte Verbrennungen, Brandwun den, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seinen genannt:

Schwein: Coli-diarrhoe, Enterotoxämie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz-und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutische geeigneten Trägerstoffen eine oder mehrere erfindungsgemäße Verbindungen enthalten oder die aus einem oder mehreren erfindungsgemäßen Wirkstoffen bestehen, sowie Verfahren zur Hertstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegt, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Phillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie (a) Füll-und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium-und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit-und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs-und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber-und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human-als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der

Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte einiger der erfindungsgemäßen Verbindungen im Vergleich zu Imipenem angegeben.

Antibakterielle Wirkung:

Das Agar-Lösungsverfahren wurde zur Bestimmung der antibakteriellen Wirkung verwendet, wobei ein Iso-Sensitest-Agar verwendet wurde und die minimale Hemmkonzentration (MHK) der Probe wurde durch µg/ml des Nährbodens ausgedrückt (Tabelle).

| Keim | Verbindung aus Bsp. 5 | Verbindung aus Bsp. 6 | Imipenem |
|---|---|---|---|
| Proteus mir. 1235 | 1 | 0,5 | 4 |
| Staph. aur. 1756 | 4 | 1 | 16 |
| Staph. aur. 133 | $\leq$ 0,0625 | $\leq$ 0,0625 | $\leq$ 0,0625 |
| Staph. aur. 25022 | $\leq$ 0,0625 | $\leq$ 0,0625 | $\leq$ 0,0625 |
| Staph. e. 25 185 | 0,125 | $\leq$ 0,0625 | $\leq$ 0,0625 |
| Strept. faec. 27101 | 2 | 0,5 | 4 |
| Strept. faec. 113 | 2 | 1 | 0,5 - 1 |
| Enterococc. 9790 | 2 | 0,5 | 4 |
| Enterococc. 27158 | 1 | 1 | 0,5 - 1 |

Herstellungsbeispiele

Beispiel 1

(5R, 6S)-3-Diphenylphosphonyloxy-6-[(IR)-1-hydroxyethyl]-7-oxo-1-azabicyclo[3.2.0]hept-2-en-2-carbonsäure-p-nitro-benzylester

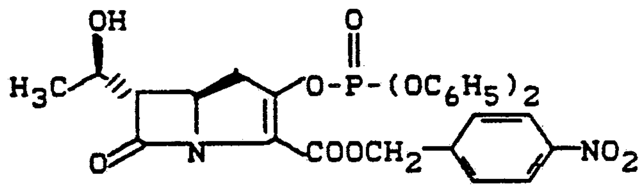

Zu einer auf 0°C gekühlten Lösung von 3,48 g (10.0 mmol) (2R,5R,6S)-3,7-Dioxi-6-[(IR)-1-hydroxyethyl]-1-azabicyclo-[3.2.0]heptan-2-carbonsäure-p-nitrobenzylester [T.N. Salzmann et al., J. Am. Chem. Soc. 102, 6163 (1980)] in 40 ml wasserfreiem Acetonitril tropfte man gleichzeitig innerhalb von 5 min 1,82 ml (10,5 mmol) N,N-Diisopropylethylamin und 2,18 ml (10,5 mmol) Phosphorsäurediphenylesterchlorid. Die Mischung wurde 30 min bei 0°C gerührt und dann in ein Gemisch aus kalter $NaHCO_3$-Lösung und Ethylacetat gegossen. Man extrahierte mit Ethylacetat, wusch die organischen Extrakte mit $NaHCO_3$-Lösung und trocknete über $MgSO_4$. Nach Abdampfen des Lösemittels im Vakuum und Trocknen des Rückstandes im Hochvakuum erhielt man die Titelverbindung als hellen Schaum, welcher direkt weiter umgesetzt wurde.
$R_f$ : 0,45 (Toluol : Ethylacetat 3:7)
IR (KBr): 3450, 1782, 1729, 1643, 1590, 1526, 1490, 1350 $cm^{-1}$
$^1$H-NMR (200 MHz, $CDCl_3$): $\delta$ = 1,34 (d, J = 6,5 Hz, 3H, $CH_3$CHOH), 3.25 (m, 3H, H-4, H-4', H-6), 4.25 (m, 2H, H-5, $CH_3$CHOH), 5,25 und 5,41 (AB, J = 13 Hz, 2H, $COOCH_2$), 7,2 - 7,5 (m, 10 H, Ph), 7,59 und 8,17 (AB, J = 8,5 Hz, 4H, p-$NO_2$-$C_6H_4$).

Beispiel 2

(5R,6S)-3-(6-Amino-2-benzthiazolylthio)-6-[(IR)-1-hydroxyethyl]-7-oxo-1-azabicyclo[3.2.0]hept-2-en-2-carbonsäure-p-nitrobenzylester

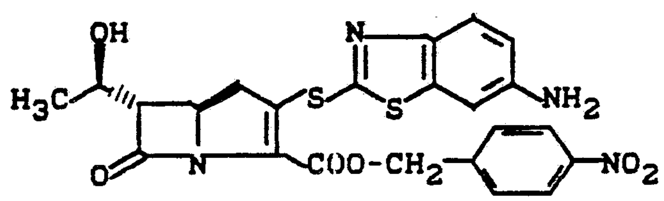

Zu einer auf -40°C gekühlten Lösung von 1,74 g (3,0 mmol) (5R,6S)-3-Diphenylphosphonyloxy-6-[(IR)-1-hydroxyethyl]-7-oxo-1-azabicyclo[3.2.0]hept-2--en-2-carbonsäure-p-nitrobenzylester in 6 ml wasserfreiem Dimethylformamid tropfte man 0,55 ml (3,15 mmol) N,N-Diisopropylethylamin und gab anschließend 0,57 g (3,15 mmol) 6-Amino-2-mercapto-benzthiazol zu. Die dabei erhaltene Suspension durfte sich innerhalb von 2 h auf 0°C erwärmen und wurde danach 42 h bei 0°C gerührt, wobei eine klare Lösung entstand. Zur Aufarbeitung goß man in ein Gemisch aus Eis, $NaHCO_3$-Lösung und Ethylacetat, extrahierte mit Ethylacetat, wusch die orga nischen Extrakte mit kalter $NaHCO_3$-Lösung (2 x) und trocknete über $MgSO_4$. Nach Abdampfen des Lösemitteln im Vakuum und Chromatographie des Rückstandes an 45 g Kieselgel (Toluol : Ethylacetat 2:3) erhielt man 465 mg (30% der Theorie) der Titelverbindung als helle Kristalle. Schmp.: 190°C (Zers.)
$R_f$ : 0,12 (Toluol : Ethylacetat 1:1)
IR (KBr): 1766, 1691, 1628, 1602, 1575, 1518, 1338 $cm^{-1}$
$^1$H-NMR (200 MHz, DMSO): $\delta$ = 1,06 (d, J = 6,5 Hz, 3H, $CH_3$CHOH), 2.94 und 3,15 (ABd, J = 18,5 Hz, 10 Hz, 2H, H-4), 3,45 (dd, J = 6 Hz, 2,5 Hz, 1H, H-6), 3,94 (dq, J = 6,5 Hz, 6 Hz, 1H, $CH_3$CHOH), 4,17 (dt, 10 Hz, 2,5 Hz, 1H, H-5), 5,04 (d, J = Hz, 1H, OH), 5,40 und 5,54 (AB, J = 14,5 Hz, 2H, $COOCH_2$), 5,72 (bs, 2H, $NH_2$), 6,78 (dd, J = 9 Hz, 2 Hz, 1H, H-5'), 7,10 (d, J = 2 Hz, 1H, H-7'), 7,75 (d, J = 9 Hz, 1H, H-4'), 7,78 und 8,31 (AB, J = 9 Hz, 4H, p-$NO_2$-$C_6H_4$).

Beispiel 3

(5R,6S)-3-(6-Formylamino-2-benzthiazolylthio)-6-[(IR)-1-hydroxyethyl]-7-oxo-1-azabicyclo[3.2.0]hept-2-en-2-carbonsäure-p-nitrobenzylester

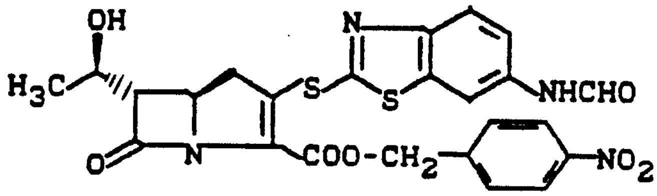

Verfahrensvariante A:

Wie für Beispiel 2 beschrieben erhielt man aus 1,08 g (2,0 mmol) (5R,6S)-3-Diphenylphosphonyloxy-6-[(IR)-1-hydroxyethyl]-7-oxo-1-azabicyclo[3.2.0]hept-2-en-2-carbonsäure-p-nitrobenzylester und 0,38 g (2.1 mmol) 6-Formylamino-2-mercapto-benzthiazol nach 42 h bei 0°C (klare Lösung nach 45 min) und Chromatographie des Rohprodukts an 77 g Kieselgel (Toluol : Ethylacetat 1:4) 376 mg (35% der Theorie) der Titelverbindung als farblose Kristalle.
Schmp.: 174°C (Zers.)
$R_f$ = 0,17 (Toluol : Ethylacetat 1:4)
IR (KBr) 3442, 1783, 1688, 1645, 1607, 1557, 1526, 1338 $cm^{-1}$
$^1$H-NMR (200 MHz, DMSO): $\delta$ = 1,08 (d, J = 6,5 Hz, 3H, $CH_3$CHOH), 3,15 und 3,38 (m, 2H, H-4), 3,48 (dd, J= 6 Hz, 3 Hz, 1H, H-6), 3,95 (dq, J = 6,5 Hz, 6 Hz, 1H, $CH_3$CHOH), 4,21 (dt, J = 10 Hz, 3 Hz, 1H, H-5), 5,07 (d, J = 5 Hz, 1H, OH), 5,41 und 5,56 (AB, J= 14 Hz, 2H, $COOCH_2$) 7,17 (dd, J = 9,5 Hz, 1,5 Hz, 1H, H-5'), 7,78 und 8,30 (AB, J = 9 Hz, 4H, p-$NO_2$-$C_6H_4$), 8,06 (d, J = 9,5 Hz, 1H, H-4'), 8,41 (bs, 1H, NH CHO), 8,58 (d, J = 1,5 Hz, 1H, H-7'), 10,60 (bs, 1H, NHCHO).

Verfahrensvariante B:

Eine auf 0°C gekühlte Lösung von 571 mg (1,64 mmol) (2R,5R,6S)-3,7-Dioxo-6-[(IR)-1-hydroxyethyl]-1-azabicyclo-[3.2.0]heptan-2-carbonsäure-p-nitrobenzylester [T.N. Salzmann et al., J. Am. Chem. Soc. 102, 6163 (1980)] in 8 ml wasserfreiem Acetonitril wurde innerhalb von 10 min gleichzeitig mit 0,3 ml (1,73 mmol - 1,05 equiv.) Ethyldiisopropylamin und 0,36 ml (1,73 mmol) Phosphorsäurediphenylesterchlorid versetzt. Danach rührte man 15 min bei 0°C, kühlte auf -40°C und gab nacheinander 0,32 ml (1,81 mmol - 1,1 equiv.) Ethyldiisopropylamin und 323 mg (1,81 mmol) 6-Formylamino-2-mercapto-benzthiazol zu. Die Reaktionsmischung durfte sich auf 0°C erwärmen und wurde nach Beendigung in ein kaltes Gemisch aus $NaHCO_3$-Lösung und Ethylacetat eingerührt. Die Mischung wurde mit Ethylacetat extrahiert, mit NaCl-Lösung und Wasser gewasche und über $MgSO_4$ getrocknet. Nach Abdampfen des Lösemittels im Vakuum und Chromatographie des Rückstandes an 100 g Kieselgel erhielt man 152 mg (17% der Theorie) der Titelverbindung. Die physikalischen Werte waren identische mit der nach Verfarhrensvariante A erhaltenen Substanz.

Beispiel 4

(5R,6S)-3-(6-Amino-2-benzimidazolylthio)-6-[(IR)-1-hydroxyethyl]-7-oxo-1-azabicyclo[3.2.0]hept-2-en-2-carbonsäure-p-nitrobenzylester

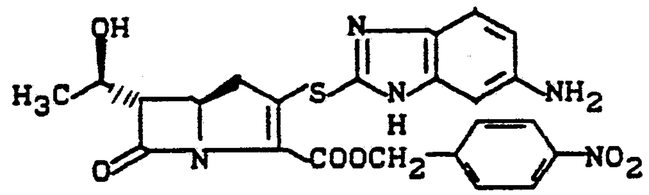

Wie für Beispiel 2 beschrieben erhielt man aus 5,80 g (10,0 mmol) (5R,6S)-3-Diphenylphosphonyloxy-6-[(IR)-1-hydroxyethyl]-7-oxo-1-azabicyclo[3.2.0]hept-2-en-2-carbonsäure-p-nitrobenzylester und 1,74 g (10,5 mmol) 6-Amino-2-mercapto-benzimidazol nach 67 h bei 0°C und Chromatographie des Rohproduktes an 600 g Kieselgel (Ethylacetat : Acetonitril 85:15) 465 mg (9,5% der Theorie) der Titelverbindung als farblose Kristalle.
Schmp.: 168°C (Zers.)
$R_f$ = 0,27 (Ethylacetat : Acetonitril 4:1)
IR (KBr) 1756, 1740, 1723, 1661, 1629, 1568, 1520, 1480, 1349 $cm^{-1}$
$^1$H-NMR (200 MHz, DMSO): $\delta$ = 1,12 (d, J = 6,5 Hz, 3H, $\underline{CH_3}CHOH$), 2,76 (m, 2H, H-4), 2,90 (dd, J = 7 Hz, 2 Hz, 1H, H-6), 3,88 (dq, J = 7 Hz, 6,5 Hz, 1H, $CH_3\underline{CHOH}$), 4,00 (dt, J = 6,5 Hz, 2 Hz, 1H, H-5), 4,14 (bs, 2H, $NH_2$), 4,95 (d, J = 4 Hz, 1H, OH), 5,19 (s, 2H, $\overline{COOCH_2}$), 7,02 (d, J = 9 Hz, 1H, H-4'), 7,14 (dd, J = 9 Hz, 1,5 Hz, 1H, H-5'), 7,64 und 8,12 (AB, J = 9,5 Hz, 4H, p-$NO_2$-$C_6H_4$), 7,64 (d, J= 1,5 Hz, 1H, H-7'), 10,10 (bs, 1H, NH).

Beispiel 5

(5R,6S)-3-(Amino-2-benzthiazolylthio)-6-[(IR)-1-hydroxyethyl]-7-oxo-1-azabicyclo[3.2.0]hept-2-en-2-carbonsäure

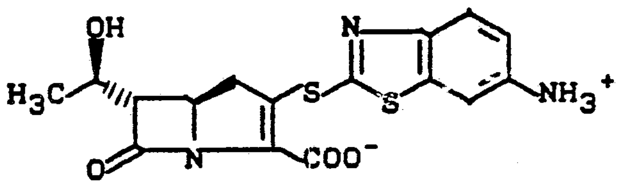

Eine Lösung von 450 mg (0,87 mmol) (5R,6S)-3-(6-Amino-2-benzthiazolylthio)-6-[(IR)-1-hydroxyethyl]-7-oxo-1-azabicyclo[3.2.0]hept-2-en-2-carbonsäure-p-nitrobenzylester in 12,5 ml eines Gemisches, bestehend aus Tetrahydrofuran und 0,1 M Phosphatpuffer pH 7 (1:1) wurde in Gegenwart von 450 mg Palladium auf Kohle (10%) 1,5 h lang hydrogenolysiert. Danach wurde der Katalysator durch Filtration über Kieselgur abgetrennt, das Tetrahydrofuran im Vakuum entfernt und die zurückbleibende Lösung auf pH 7 gestellt. Die wässrige Lösung wurde 2 mal mit Ethylacetat extrahiert, im Vakuum auf ca. 10 ml eingeengt und auf eine Säule mit 120 ml Diaion HP 20 geladen. Es wurde mit Wasser, welches einen steigenden Anteil an Acetonitril anthielt, eluiert. Die das Produkt enthaltenden Fraktionen wurden gesammelt, durch einen 0,2 $\mu$ Filter filtriert und gefriergetrocknet. Man erhielt 253 mg (73% der Theorie) der Titelverbindung als farbloses Lyophilisat. $R_f$ = 0,26 (Acetonitril : Wasser = 9:1) in einer Reinheit von 97% (HPLC).
IR (KBr) 3420, 1763, 1601, 1477, 1403 $cm^{-1}$
$^1$H-NMR (200 MHz, $D_2O$): $\delta$ = 1,19 (d, J = 7 Hz, 3H, $\underline{CH_3}CHOH$), 2,86 (m, 2H, H-4), 3,31 (dd, J = 6 Hz, 2,5 Hz, 1H, H-6), 4,13 (dt, J = 7 Hz, 2,5 Hz, H-5), 4,18 (dq, J = 7 Hz, 7 Hz, $CH_3\underline{CH}OH$), zusammen 2H), 7,05 (dd, J = 9 Hz, 1,5 Hz, 1H, H-5'), 7,25 (d, J= 1,5 Hz, 1H, H-7'), 7,78 (d, J = 9 Hz, 1H,H-4').

Beispiel 6

Natrium(5R,6S)-3-(6-formylamino-2-benzthiazolylthio)-6-[(IR)-1-hydroxyethyl]-7-oxo-1-azabicyclo[3.2.0]hept-2-en-2-carboxylat

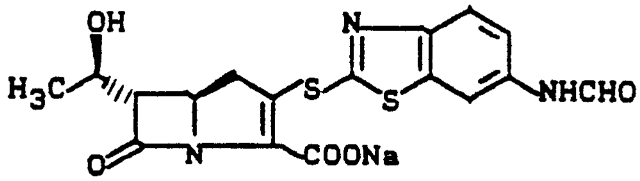

Wie für Beispiel 5 beschrieben erhielt man aus 2,73 g (5,05 mmol) (5R,6S)-3-(6-Formylamino-2-benzthiazolylthio)-6-[(IR)-1-hydroxyethyl]-7-oxo-1-azabicyclo[3.2.0]hept-2-en-2-carbonsäure-p-nitrobenzylester 1.95 g (90% der Theorie) der Titelverbindung als farbloses Lyophilisat.
$R_f$ = 0,3 (Acetonitril : $H_2O$ 9:1), in einer Reinheit von 96% (HPLC).
IR (KBr) 3403, 1764, 1684, 1601, 1402 $cm^{-1}$
$^1$H-NMR (200 MHz, $D_2O$): $\delta$ = 1,09 (d, J = 7 Hz, 3H, <u>CH</u>$_3$CHOH), 2,80 und 2,85 (bs, 2H, H-4), 3,28 (m, 1H, H-5), 4,06 (m, 2H, H-5, CH$_3$<u>CH</u> OH), 7,30 (d, J = 9 Hz, 1H, H-5'), 7,63 (d, J = 9 Hz, 1H, H-4'), 7,94 (s, 1H, H-7'), 8,20 (s, 1H, CHO).

Beispiel 7

(5R,6S)-3-(6-Amino-2-benzimidazolylthio)-6-[(IR)-1-hydroxyethyl]-7-oxo-1-azabicyclo[3.2.0]hept-2-en-2-carbonsäure

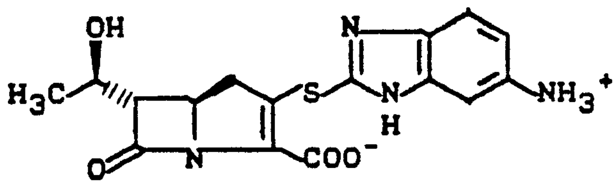

Wie für Beispiel 5 beschrieben erhielt man aus 550 mg (1,1 mmol) (5R,6S)-3-(6-Amino-2-benzimidazolylthio)-6-[(IR)-1-hydroxyethyl]-7-oxo-1-azabicyclo[3.2.0]hept-2-en-2-carbonsäure-p-nitrobenzylester 384 mg (97% der Theorie) der Titelverbindung als farbloses Lyophilisat.
$R_f$ = 0,20 (Acetonitril : $H_2O$ 9:1), in einer Reinheit von 98% (HPLC).
IR (KBr) 3440, 1760, 1659, 1627, 1477, 1385 $cm^{-1}$
$^1$H-NMR (300 MHz, $D_2O$): $\delta$ = 1,25 (d, J = 6,5 Hz, 2H, <u>CH</u>$_3$CHOH), 2,87 (dd, J = 15 Hz, 7,5 Hz, 1H, H-4), 2,97 (dd, J = 15 Hz, 8 Hz, 1H, H-4), 3,18 (dd, J = 6,5 Hz, 2,5 Hz, 1H, H-6), 4,2 (m, 2H, CH$_3$<u>CH</u>OH, H-5), 7,11 (dd, J = 9 Hz, 1 Hz, 1H, H-5'), 7,20 (d, J = 9 Hz, 1H, H-4'), 7,37 (d, J = 1 Hz, 1H, H-7').

**Ansprüche**

1. Benzazolylthio-carbapeneme der allgemeinen Formel (I)

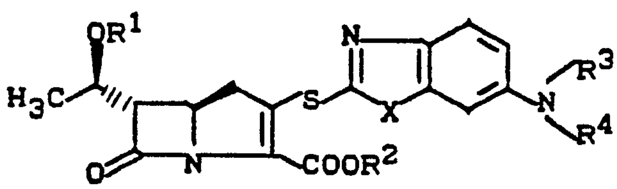

(I)

in welcher

$R^1$ -für Wasserstoff oder
-für eine Hydroxyschutzgruppe steht,
$R^2$ -für Wasserstoff oder
-für eine Carboxyschutzgruppe oder
-für einen in vivo abspaltbaren Esterrest steht,
$R^3$ -für Wasserstoff oder
-für $C_1$-$C_{10}$-Alkyl, $C_6$-$C_{12}$-Aryl oder $C_7$-$C_{14}$-Aralkyl steht,
$R^4$ -für Wasserstoff oder
-für $C_1$-$C_{10}$-Alkyl, $C_6$-$C_{12}$-Aryl oder $C_7$-$C_{14}$-Aralkyl steht, oder
-für $C_1$-$C_{10}$-Alkylsulfonyl, $C_6$-$C_{12}$-Arylsulfonyl oder $C_7$-$C_{14}$-Aralkylsulfonyl steht, oder
-für eine Aminoschutzgruppe steht, oder
-für eine Gruppe der Formel

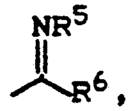, 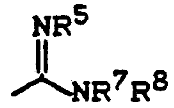 oder 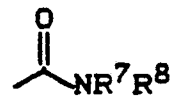 steht,

worin
$R^5$, $R^6$, $R^7$ und $R^8$ gleich oder verschieden sind und
-Wasserstoff, $C_1$-$C_{10}$-Alkyl, $C_6$-$C_{12}$-Aryl oder $C_7$-$C_{14}$-Aralkyl bedeuten und
X - für O, S, NH oder $NCH_3$ steht
und deren Salze.

2. Verbindungen nach Anspruch 1, in denen die Aminoschutzgruppe für Vinyl, Allyl, tert.Butoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, 4-Methoxybenzyloxycarbonyl, Formyl, Benzoyl, Acetyl, Ethylcarbonyl, Chloracetyl, Trichloracetyl, Trifluoroacetyl, Methyloxycarbonyl, Allyloxycarbonyl, Trimethylsilyl, Triethylsilyl, Triphenylsilyl, tert.Butyl-dimethylsilyl, Methyldiphenylsilyl, 2,4-Dimethoxybenzyl, 2,4-Dimethoxybenzyloxycarbonyl, 4-Methoxybenzyl, 4-(Methoxymethyloxy)phenyl, Bis-(4-Methoxyphenyl)methyl, tert.Butoxycarbonylmethyl, Allyloxycarbonylmethyl, Methoxymethyl, Methylthiomethyl, Methoxyethoxymethyl, [2-(Trimethylsilyl)ethoxy]methyl oder 2-(Methylthiomethoxy)ethoxycarbonyl steht.

3. Verbindungen nach Anspruch 1, in denen die Hydroxyschutzgruppe für Trimethylsilyl, Triethylsilyl, Triisopropylsilyl, tert.Butyl-dimethylsilyl, Tri phenylsilyl, Trimethylsilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, tert.Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]methyl, 2-(Methylthiomethoxy)-ethoxycarbonyl, Tetrahydropyranyl oder Benzoyl und die Carboxyschutzgruppe für Methyl, Ethyl, tert.-Butyl, Decyl, 2-Chlorethyl, 2,2,2-Trichlorethyl, Cyanoethyl, Diphenylmethyl, Triphenylmethyl, Acetoxymethyl, Allyl, Benzyl, 4-Methoxyphenyl, 4-Nitrobenzyl, 2-Nitrobenzyl, 4-Methoxybenzyl, 2,4-Dimethoxybenzyl, Trimethylsilylethyl, Trimethylsilyl, tert.Butyl-dimethylsilyl, Acetonyl, 1-Phenoxyethyl oder 2-Methyl-2-propenyl steht.

4. Verbindungen nach Anspruch 1, in denen $R^2$ für einen Rest aus der Gruppe bestehend aus

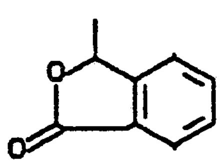, 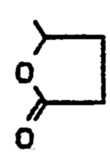, 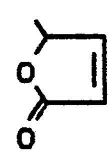,

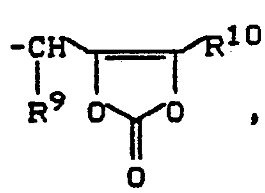, 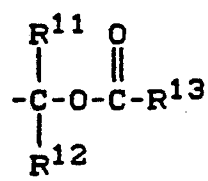 oder

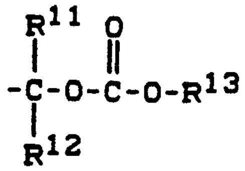

steht, worin
$R^9$ und $R^{10}$ gleich oder verschieden sind und
-für Wasserstoff, Phenyl oder
-für $C_1$-$C_4$-Alkyl, bevorzugt für Methyl stehen,
$R^{11}$ und $R^{12}$ gleich oder verschieden sind und
-für Wasserstoff oder
-für $C_1$-$C_4$-Alkyl, bevorzugt Methyl stehen und
$R^{13}$ -für $C_1$-$C_6$-Alkyl, bevorzugt für $C_1$-$C_4$-Alkyl steht.

5. Verbindungen nach Anspruch 1 der allgemeinen Formel (I)
in welcher
$R^1$ -für Wasserstoff steht, oder
-für eine Hydroxyschutzgruppe aus der Reihe Trimethylsilyl, Triethylsilyl, Triisopropylsilyl, tert.Butyl-dimethylsilyl, Triphenylsilyl, Trimethyslilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, tert.Butoxycarbonyl, Allyloxycarbonyl, 4-Methoxybenzyl, 4-Methoxybenzyloxycarbonyl, Formyl, Acetyl, Trichloracetyl, 2,2,2,-Trichlorethoxycarbonyl, 2,4-Dimethoxybenzyloxycarbonyl, Methoxymethyl oder Methoxyethoxymethyl steht,
$R^2$ -für Wasserstoff steht, oder
-für Methyl, Ethyl, tert.Butyl, 2-Chlorethyl, 2,2,2-Trichlorethyl, Cyanoethyl, Diphenylmethyl, Triphenylmethyl, Acetoxymethyl, Allyl, Benzyl, 4-Methoxybenzyl, 2,4-Dimethoxybenzyl, 1-Phenoxyethyl, 2-Methyl-2-propenyl, 4-Nitrobenzyl, 2-Nitrobenzyl, Trimethylsilylethyl oder tert.Butyl-dimethylsilylethyl steht, oder
-für einen Rest der Formel

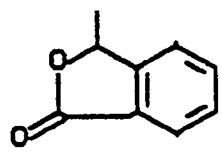 , 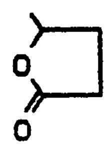 , 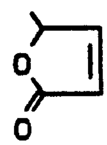 ,

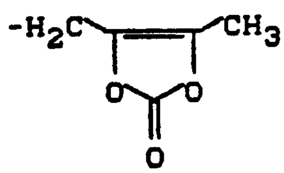 ,

$-CH_2-OCO-C(CH_3)_3$, $-CH(CH_3)-OCOOC_2H_5$ oder $-CH_2-OCOCH_3$ steht,
$R^3$ -für Wasserstoff oder
-für $C_1$-$C_6$-Alkyl steht, oder
-für Phenyl oder Benzyl steht,
$R^4$ -für Wasserstoff steht, oder
-für $C_1$-$C_6$-Alkyl steht, oder
-für Phenyl oder Benzyl steht, oder
-für $C_1$-$C_4$-Alkylsulfonyl, Phenylsulfonyl oder Benzylsulfonyl steht, oder
-für eine Aminoschutzgruppe aus der Reihe Allyl, tert.Butoxycarbonyl, Benzyl, Benzyloxycarbonyl, 4-Nitrobenzyl, 4-Nitrobenzyloxycarbonyl, 4-Methoxybenzyloxycarbonyl, Formyl, Acetyl, Trichloracetyl, 4-Methoxyphenyl, 4-Methoxybenzyl, 2,4-Dimethoxybenzyl, 2-Nitrobenzyloxycarbonyl, tert.Butyl-dimethylsilyl, Methyldiphenylsilyl, Trimethylsilyl oder Trifluoracetyl steht, oder
-für eine Gruppe der Formel

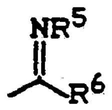, 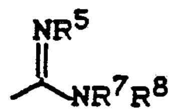 oder 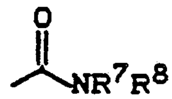 steht,

worin
$R^5$, $R^6$, $R^7$ und $R^8$ gleich oder verschieden sind und
-Wasserstoff oder $C_1$-$C_6$-Alkyl, Phenyl oder Benzyl bedeuten und
X - für O oder S steht, oder
-für NH oder $NCH_3$ steht
und deren Salze.

6. Verbindungen nach Anspruch 1 der allgemeinen Formel (I)
in welcher
$R^1$ -für Wasserstoff steht, oder
-für Trimethylsilyl, Triphenylsilyl, Trimethylsilylethoxycarbonyl, tert.Butyldimethylsilyl, 2-Nitrobenzyloxycarbonyl, 4-Nitrobenzyloxycarbonyl, Allyloxycarbonyl oder Formyl steht,
$R^2$ -für Wasserstoff steht, oder
-für Methyl, Ethyl, tert.Butyl, 2,2,2-Trichlorethyl, Allyl, Acetoxymethyl, 4-Nitrobenzyl, 2-Nitrobenzyl, 4-Methoxybenzyl, Benzyl oder Trimethylsilylethyl steht, oder
-für einen Rest der Formel

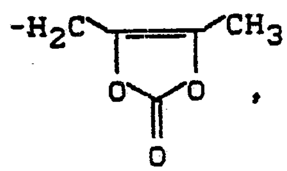 ,

$-CH(CH_3)-OCOOC_2H_5$ oder $-CH_2-OCO-C(CH_3)_3$ steht,
$R^3$ -für Wasserstoff, $C_1$-$C_4$-Alkyl oder Phenyl steht,
$R^4$ -für Wasserstoff oder
-für $C_1$-$C_4$-Alkyl, Phenyl, tert.Butyloxycarbonyl, Benzyloxycarbonyl, Acetyl, Formyl oder Ethylcarbonyl steht, oder
-für eine Gruppe der Formel

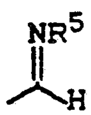 , 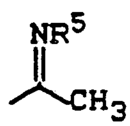 , 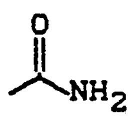 oder 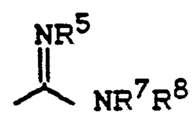

steht,
worin
$R^5$, $R^7$ und $R^8$ gleich oder verschieden sind und - Wasserstoff, Methyl oder Phenyl bedeuten, und
X - für S, NH oder $NCH_3$ steht
und deren Salze.

7. Verfahren zur Herstellung der Benzazolylthio-Carbapeneme der allgemeinen Formel (I) nach Anspruch 1 und deren Salze, dadurch gekennzeichnet, daß man
Ketoester der allgemeinen Formel (II)

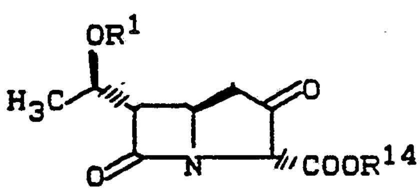 (II)

in welcher

$R^1$ -die im Anspruch 1 angegebene Bedeutung hat und
$R^{14}$ -für eine Carboxyschutzgruppe oder
-für einen in vivo abspaltbaren Esterrest steht und
Thiole der allgemeinen Formel (III)

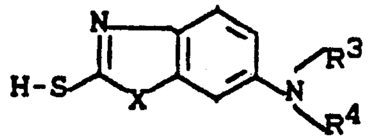

(III)

in welcher
$R^3$, $R^4$ und X die in Anspruch 1 angegebene Bedeutung haben,
in inerten Lösemitteln in Gegenwart von Basen mit Hilfsstoffen umsetzt,
gegebenenfalls Schutzgruppen abspaltet
und gegebenenfalls die gewünschten Salze herstellt oder die Salze in die freien Verbindungen überführt.

8. Benzazolylthio-carbapeneme der allgemeinen Formel (I)

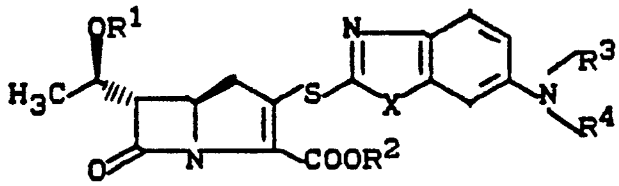

(I)

in welcher
$R^1$ -für Wasserstoff der
-für eine Hydroxyschutzgruppe steht,
$R^2$ -für Wasserstoff oder
-für eine Carboxyschutzgruppe oder
-für einen in vivo abspaltbaren Esterrest steht,
$R^3$ -für Wasserstoff oder
-für $C_1$-$C_{10}$-Alkyl, $C_6$-$C_{12}$-Aryl oder $C_7$-$C_{14}$-Aralkyl steht,
$R^4$ -für Wasserstoff oder
-für $C_1$-$C_{10}$-Alkyl, $C_6$-$C_{12}$-Aryl oder $C_7$-$C_{14}$-Aralkyl steht, oder
-für $C_1$-$C_{10}$-Alkylsulfonyl, $C_6$-$C_{12}$-Arylsulfonyl oder $C_7$-$C_{14}$-Aralkylsulfonyl steht, oder
-für eine Aminoschutzgruppe steht, oder
-für eine Gruppe der Formel

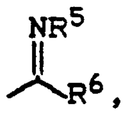 , 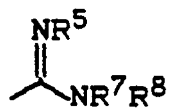 oder 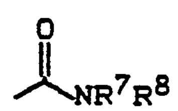 steht,

worin
$R^5$, $R^6$, $R^7$ und $R^8$ gleich oder verschieden sind und
-Wasserstoff, $C_1$-$C_{10}$-Alkyl, $C_6$-$C_{12}$-Aryl oder $C_7$-$C_{14}$-Aralkyl bedeuten und
X - für O, S, NH oder $NCH_3$ steht
und deren Salze, zur Anwendung in einem Verfahren zur therapeutischen Behandlung des menschlichen oder tierischen Körpers.

9. Arzneimittel, enthaltend Benzazolyltion-carbapeneme der allgemeinen Formel (I)

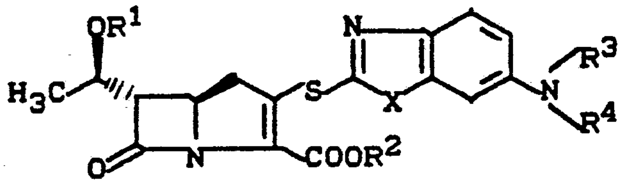

(I)

in welcher
$R^1$ -für Wasserstoff oder
-für eine Hydroxyschutzgruppe steht,
$R^2$ -für Wasserstoff oder
-für eine Carboxyschutzgruppe oder
-für einen in vivo abspaltbaren Esterrest steht,
$R^3$ -für Wasserstoff oder
-für $C_1$-$C_{10}$-Alkyl, $C_6$-$C_{12}$-Aryl oder $C_7$-$C_{14}$-Aralkyl steht,
$R^4$ -für Wasserstoff oder
-für $C_1$-$C_{10}$-Alkyl, $C_6$-$C_{12}$-Aryl oder $C_7$-$C_{14}$-Aralkyl steht, oder
-für $C_1$-$C_{10}$-Alkylsulfonyl, $C_6$-$C_{12}$-Arylsulfonyl oder $C_7$-$C_{14}$-Aralkylsulfonyl steht, oder
-für eine Aminoschutzgruppe steht, oder
-für eine Gruppe der Formel

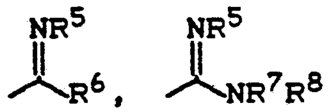 oder 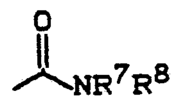 steht,

worin
$R^5$, $R^6$, $R^7$ und $R^8$ gleich oder verschieden sind und
-Wasserstoff, $C_1$-$C_{10}$-Alkyl, $C_6$-$C_{12}$-Aryl oder $C_7$-$C_{14}$-Aralkyl bedeuten und
X - für O, S, NH oder $NCH_3$ steht
und deren Salze.

10. Verwendung von Benzazolylthio-carbapenemen der allgemeinen Formel (I)

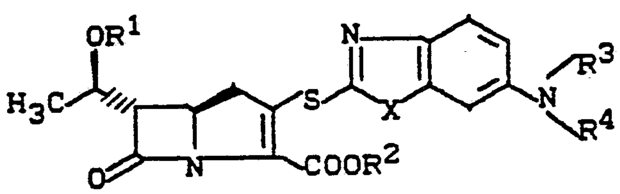

(I)

in welcher
$R^1$ -für Wasserstoff oder
-für eine Hydroxyschutzgruppe steht,
$R^2$ -für Wasserstoff oder
-für eine Carboxyschutzgruppe oder
-für einen in vivo abspaltbaren Esterrest steht,
$R^3$ -für Wasserstoff oder
-für $C_1$-$C_{10}$-Alkyl, $C_6$-$C_{12}$-Aryl oder $C_7$-$C_{14}$-Aralkyl steht,
$R^4$ -für Wasserstoff oder
-für $C_1$-$C_{10}$-Alkyl, $C_6$-$C_{12}$-Aryl oder $C_7$-$C_{14}$-Aralkyl steht, oder
-für $C_1$-$C_{10}$-Alkylsulfonyl, $C_6$-$C_{12}$-Arylsulfonyl oder $C_7$-$C_{14}$-Aralkylsulfonyl steht, oder
-für eine Aminoschutzgruppe steht, oder
-für eine Gruppe der Formel

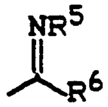, 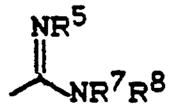 oder 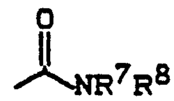 steht,

worin

$R^5$, $R^6$, $R^7$ und $R^8$ gleich oder verschieden sind und

-Wasserstoff, $C_1$-$C_{10}$-Alkyl, $C_6$-$C_{12}$-Aryl oder $C_7$-$C_{14}$-Aralkyl bedeuten und

X - für O, S, NH oder $NCH_3$ steht

und deren Salzen, bei der Herstellung von Arzneimitteln.

Europäisches Patentamt

# EUROPÄISCHER RECHERCHENBERICHT

Nummer der Anmeldung

EP 87116840.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 182 213 (SUMITOMO)<br>* Ansprüche *<br>-- | 1,7-10 | C 07 D 487/04<br>A 61 K 31/395 |
| A | EP - A1 - 0 170 073 (MERCK)<br>* Ansprüche *<br>---- | 1,8-10 | |
| | | | RECHERCHIERTE SACHGEBIETE (Int. Cl.4) |
| | | | C 07 D 487/00 |

Der vorliegende Recherchenbericht wurde für alle Patentansprüche erstellt.

| Recherchenort | Abschlußdatum der Recherche | Prüfer |
|---|---|---|
| WIEN | 03-02-1988 | JANISCH |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82